(19) Europäisches Patentamt
European Patent Office
Office européen des brevets

(11) Publication number: 0 345 751 A1

(12) # EUROPEAN PATENT APPLICATION

(21) Application number: 89110278.2

(51) Int. Cl.4: A61K 31/70

(22) Date of filing: 07.06.89

(30) Priority: 08.06.88 US 203859

(43) Date of publication of application:
13.12.89 Bulletin 89/50

(84) Designated Contracting States:
AT BE CH DE ES FR GB GR IT LI LU NL SE

(71) Applicant: MERRELL DOW PHARMACEUTICALS INC.
2110 East Galbraith Road
Cincinnati Ohio 45215-6300(US)

(72) Inventor: Sunkara, Sai Prasad
9629 Linfield Drive
Cincinnati Ohio 45242(US)
Inventor: Farr, Robert A.
2960 Maureen Court
Loveland Ohio 45140(US)

(74) Representative: Vossius & Partner
Siebertstrasse 4 P.O. Box 86 07 67
D-8000 München 86(DE)

(54) **Anti-retroviral diflourinated nucleosides.**

(57) Certain difluorinated nucleosides are disclosed to be effective in treating retroviral infections including HIV infections and are thus useful in the treatment of AIDS and ARC.

EP 0 345 751 A1

Xerox Copy Centre

## ANTI-RETROVIRAL DIFLUORINATED NUCLEOSIDES

This invention relates to the use of certain difluorinated nucleosides in the treatment of retroviral infections including HIV infections.

A great deal of research is currently underway to develop treatments and cures for viral infections in humans and in animals. Notably the incidence of AIDS and ARC in humans is increasing at an alarming rate. The five year survival rate for those with AIDS is dispiriting and AIDS patients, whose immune systems have been seriously impaired by the infection, suffer from numerous opportunistic infections including Kaposi's sarcoma and Pneumocystis carninii pneumonia. No cure for AIDS is known and current treatments are largely without adequate proof of efficacy and have numerous untoward side effects. Fear of the disease has resulted in social ostracism of and discrimination against those having or suspected of having the disease.

Retroviruses are a class of ribonucleic acid (RNA) viruses that replicate by using reverse transcriptase to form a strand of complementary DNA (cDNA) from which a double stranded, proviral DNA is produced. This proviral DNA is then randomly incorporated into the chromasomal DNA of the host cell making possible viral replication by later translation of the integrated DNA containing the viral genome.

Many of the known retroviruses are oncogenic or tumor causing. Indeed the first two human retroviruses discovered, denoted human T-cell leukemia virus I and II or HTLV-I and II, were found to cause rare leukemias in humans after infection of T-lymphocytes. The third such human virus to be discovered, HTLV-III, now referred to as HIV, was found to cause cell death after infection of T-lymphocytes and has been identified as the causative agent of acquired immune deficiency syndrome (AIDS) and AIDS related complex (ARC).

Because of the special characteristics of retroviruses and their means of replication, antiviral agents in general have not been effective in the treatment of retroviral infections. Activity of a class of compounds as antiviral agents has not been predictive of the antiretroviral activity of such compounds and compounds having retroviral activity frequently are not significantly active as broad spectrum antiviral agents. For example, the compound 3′-azido-3′-deoxythymidine, commenly referred to as AZT, is highly effective in the treatment of AIDS, but this compound is almost devoid of antiviral activity other than its antiretroviral activity.

Certain difluorinated nucleosides are known from United States Patent Number 4,526,988 and have been reported to possess antiviral and anti-tumor activity. Applicants have discovered that these antiviral agents, unlike many other antiviral agents, also possess important activity in the treatment of retroviral infections and are especially useful in the treatment of AIDS.

The present invention is directed to certain difluorinated nucleoside derivatives. More particularly, it is directed to compounds having the following formula 1:

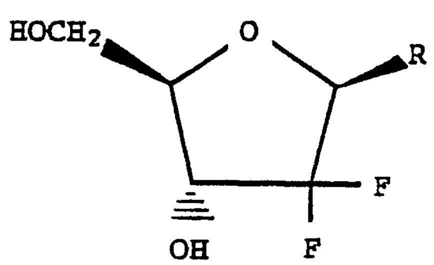

1

wherein R is a base selected from the group having one of the structural formulae 2 - 6 as follows:

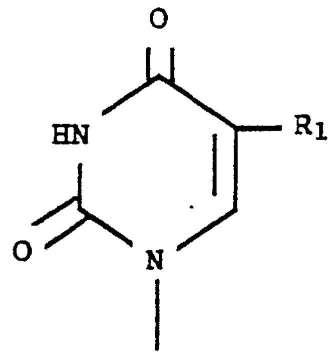

2

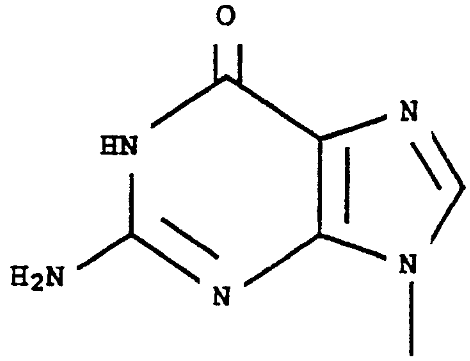

5

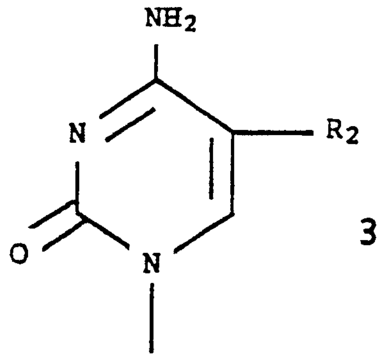

3

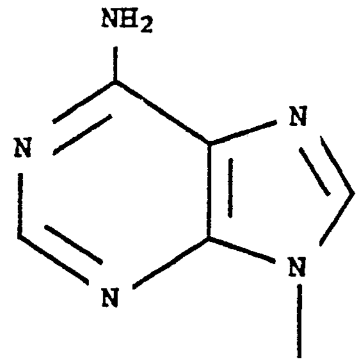

6

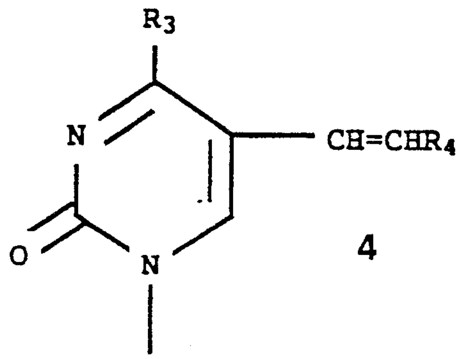

4

wherein
$R_1$ and $R_2$ is a hydrogen, methyl, bromo, fluoro, chloro, or iodo;
$R_3$ is hydroxy or amino; and
$R_4$ is chloro, bromo, or iodo;
or a pharmaceutically acceptable salt thereof.

The compounds of this invention are generally known in the art, for example, from United States Patent 4,692,434 granted September 8, 1987, and can readily be prepared as described.

The expression "a pharmaceutically acceptable acid addition salt" is intended to apply to any non-toxic organic or inorganic acid addition salt of the base compounds. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono, di, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. These salts and the base compounds can exist in either a hydrated or a substantially anhydrous form. The acid salts are prepared by standard techniques such as by dissolving the free base in aqueous or aqueous-alcohol solution or other suitable solvent containing the appropriate acid and isolating by evaporating the solution, or by reacting the free base in an organic solvent in which case

the salt separates directly or can be obtained by concentration of the solution. In general the acid addition salts of the compounds of this invention are crystalline materials which are soluble in water and various hydrophilic organic solvents and which in comparison to their free base forms, demonstrate higher melting points and an increased chemical stability.

Preferred compounds of the present invention are those wherein R is structure 2, $R_1$ = $CH_3$, and wherein R is structure 3 and $R_2$ = H are. Of those compounds wherein R is structure 4, those wherein the double bond to which the $R_4$ group is attached has trans geometry are preferred. The most preferred compound is that compound of formula 1 wherein R is structure 3, and $R_2$ is hydrogen, that is, the compound 2′-deoxy-2′,2′-difluorocytidine.

The ability of the compounds of formula 1 to act as anti-retroviral agents can be demonstrated by their ability to inhibit the growth and replication of murine leukemia virus, an oncogenic retrovirus, as determined by an *in vitro* XC plaque assay. This assay was performed according to the method of Rowe *et al.* (Virology, 1970, 42, 1136-39) as previously described by L. Hsu, *et al.* (J. Virological Methods, 1980, 1, 167-77) and T. L. Bowlin and M. R. Proffitt (J. Interferon Res., 1983, 3(1), 19-31). Mouse SC-1 cells (fibroblast) ($10^5$) were seeded into each well of 6-well cluster plates (Costar #3506) in 4 ml Minimum Essential Medium (MEM) with 10% Fetal Calf Serum (FCS). Following an 18 hour incubation period (37° C), Moloney murine leukemia virus (MoLV) was applied at a predetermined titer to give optimal (i.e. countable) numbers of virus plaques. Compounds were added 2 hours prior to addition of the virus. Three days later the culture medium was removed, the SC-1 cell monolayers were exposed to UV irradiation (1800 ergs), and rat XC cells (106) were seeded into each well in 4 ml MEM. Following an additional 3 day incubation (37° C), these cells were fixed with ethyl alcohol (95%) and stained with 0.3% crystal violet. Plaques were then counted under low magnification. The antiviral activities of various compounds of this invention are tabulated in Table 1.

TABLE 1

ANTIRETROVIRAL ACTIVITY OF 2′-DEOXY-2′,2′-DIFLUOROCYTIDINE

| Compound | Concentration (μg/ml) | Average No. of Foci/plate | % Inhibition |
|---|---|---|---|
| 2′-Deoxy-2′,2′-Difluorocytidine | 0.1 | 0 | 100 |
| 2′-Deoxy-2′,2′-Difluorocytidine | 0.01 | 0 | 100 |
| 2′-Deoxy-2′,2′-Difluorocytidine | 0.005 | 15 | 58 |
| 2′-Deoxy-2′,2′-Difluorocytidine | 0.0025 | 28 | 22 |
| AZT | 0.001 | 18 | 50 |
| Control | | 36 | -- |

The compounds of formula 1 can be used to treat a number of diseases and conditions known to be caused by retroviruses including those diseases and conditions caused by murine leukemia virus, feline leukemia virus, avian sarcoma virus, human immunodeficiency virus (HIV), HTLV-I, and HTLV-II. Those experienced in this field are readily aware of the circumstances requiring anti-retroviral therapy. Applicants consider the use of the compounds of formula 1 to treat HIV infections in humans to be of most importance. The term "patient" used herein is taken to mean mammals such as primates, including humans, sheep, horses, cattle, pigs, dogs, cats, rats and mice.

The amount of the formula 1 compound to be administered can vary widely according to the particular dosage unit employed, the period of treatment, the age and sex of the patient treated, the nature and extent of the disorder treated, and the particular compound of formula 1 selected. Moreover, compounds of formula 1 can be used in conjunction with other agents known to be useful in the treatment of retroviral diseases and agents known to be useful to treat the symptoms of and complications associated with diseases and conditions caused by retroviruses. The anti-retrovirally effective amount of a compound of formula 1 to be administered will generally range from about 15 mg/kg to 500 mg/kg. A unit dosage may contain from 25 to 500 mg of the formula 1 compound, and can be taken one or more times per day. The formula 1 compound can be administered with a pharmaceutical carrier using conventional dosage unit forms either orally or parenterally.

The preferred route of administration is oral administration. For oral administration a compound of

formula 1 can be formulated into solid or liquid preparations such as capsules, pills, tablets, troches, lozenges, melts, powders, solutions, suspensions, or emulsions. The solid unit dosage forms can be a capsule which can be of the ordinary hard- or soft-shelled gelatin type containing, for example, surfactants, lubricants, and inert fillers such as lactose, sucrose, calcium phosphate, and cornstarch. In another embodiment the compounds of this invention can be tableted with conventional tablet bases such as lactose, sucrose, and cornstarch in combination with binders such as acacia, cornstarch, or gelatin, disintegrating agents intended to assist the break-up and dissolution of the tablet following administration such as potato starch, alginic acid, corn starch, and guar gum, lubricants intended to improve the flow of tablet granulations and to prevent the adhesion of tablet material to the surfaces of the tablet dies and punches, for example, talc, stearic acid, or magnesium, calcium, or zinc stearate, dyes, coloring agents, and flavoring agents intended to enhance the aesthetic qualities of the tablets and make them more acceptable to the patient. Suitable excipients for use in oral liquid dosage forms include diluents such as water and alcohols, for example, ethanol, benzyl alcohol, and the polyethylene alcohols, either with or without the addition of a pharmaceutically acceptably surfactant, suspending agent, or emulsifying agent.

A compound of formula 1 may also be administered parenterally, that is, subcutaneously, intravenously, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,3-dioxolane-4-methanol, ethers such as poly(ethylene-glycol) 400, an oil, a fatty acid, a fatty acid ester or glyceride, or an acetylated fatty acid glyceride with or without the addition of a pharmaceutically acceptable surfactant such as a soap or a detergent, suspending agent such as pectin, carbomers, methylcellulose, hydroxypropylmethylcellulose, or carboxymethylcellulose, or emulsifying agent and other pharmaceutical adjuvants. Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum, and mineral oil. Suitable fatty acids include oleic acid, stearic acid, and isostearic acid. Suitable fatty acid esters are, for example, ethyl oleate and isopropyl myristate. Suitable soaps include fatty alkali metal, ammonium, and triethanolamine salts and suitable detergents include cationic detergents, for example, dimethyl dialkyl ammonium halides, alkyl pyridinium halides, and alkylamines acetates; anionic detergents, for example, alkyl, aryl, and olefin sulfonates, alkyl, olefin, ether, and monoglyceride sulfates, and sulfosuccinates; nonionic detergents, for example, fatty amine oxides, fatty acid alkanolamides, and polyoxyethylenepolypropylene copolymers; and amphoteric detergents, for example, alkyl-beta-aminopropionates, and 2-alkylimidazoline quarternary ammonium salts, as well as mixtures. The parenteral compositions of this invention will typically contain from about 0.5 to about 25% by weight of the formula 1 compound in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulations ranges from about 5 to about 15% by weight. The surfactant can be a single component having the above HLB or can be a mixture of two or more components having the desired HLB. Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

## EXAMPLE 1

Tablets are prepared each having the composition:

| | |
|---|---|
| 2′-deoxy-2′,2′-difluorocytidine | 250 mg |
| starch | 40 mg |
| talc | 10 mg |
| magnesium stearate | 10 mg |

## EXAMPLE 2

Capsules are prepared each having the composition:

| | |
|---|---|
| 2′-deoxy-2′,2′-difluorocytidine | 400 mg |
| talc | 40 mg |
| sodium carboxymethylcellulose | 40 mg |
| starch | 120 mg |

EXAMPLE 3

Injectable dosages forms are prepared each having the composition:

| | |
|---|---|
| 2′-deoxy-2′,2′-difluorocytidine | 0.500 g |
| polyoxyethylene sorbitan monooleate | 2.000 g |
| sodium chloride | 0.128 g |
| water for injection qs ad | 20.000 ml |

**Claims**

1. Use of compounds of the formula:

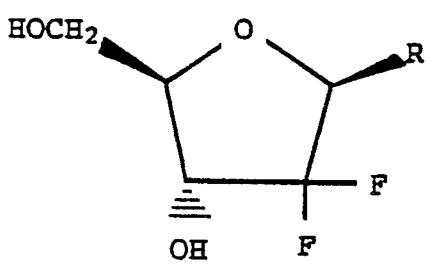

1

wherein R is a base selected from the group having the structural formulae as follows:

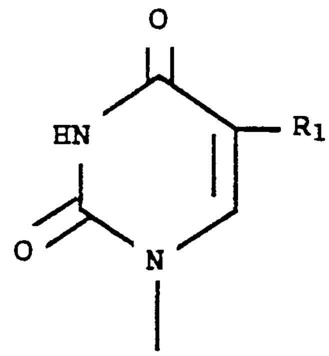

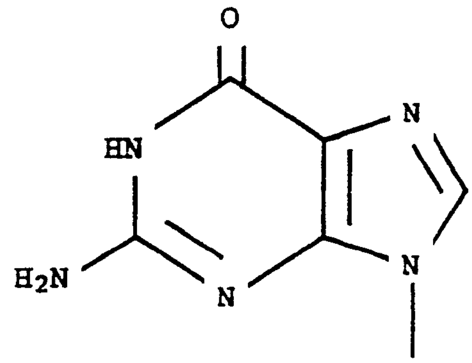

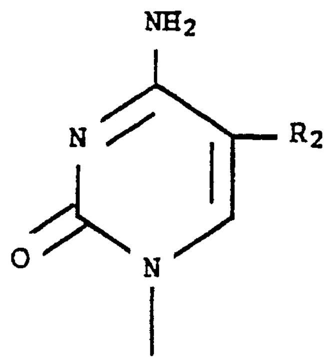

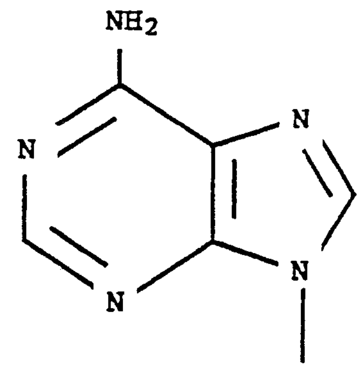

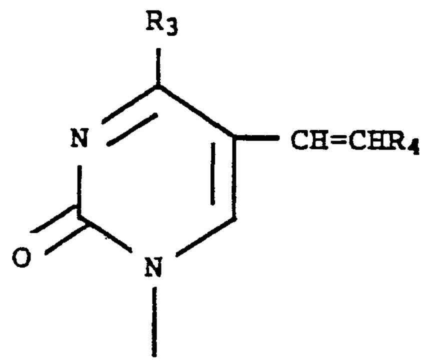

wherein
$R_1$ or $R_2$ is a hydrogen, methyl, bromo, fluoro, chloro, or iodo;
$R_3$ iS hydroxy or amino; and
$R_4$ is chloro, bromo, or iodo;
or of a pharmaceutically acceptable salt thereof for the preparation of a pharmaceutical composition for treating a retroviral infection in a patient in need thereof.

2. Use according to claim 1 wherein R is a group of the structure

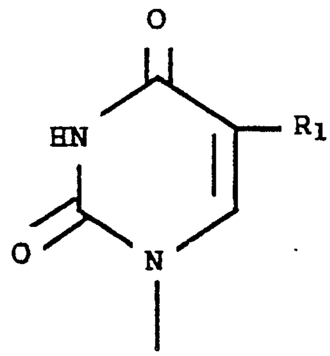

wherein $R_1$ is a hydrogen, methyl, bromo, fluoro, chloro, or iodo, or a pharmaceutically acceptable salt thereof.

3. Use according to claim 2 wherein $R_1$ is methyl or a pharmaceutically acceptable salt thereof.

4. Use according to claim 1 wherein R is a group of the structure

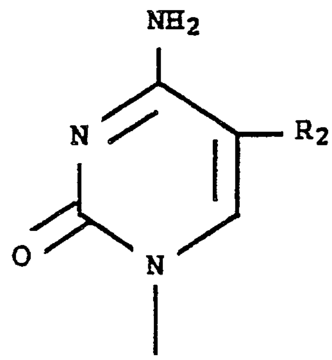

wherein $R_2$ is a hydrogen, methyl, bromo, fluoro, chloro, or iodo, or a pharmaceutically acceptable salt thereof.

5. Use according to claim 4 wherein $R_2$ is hydrogen or methyl or a pharmaceutically acceptable salt thereof.

6. Use according to claim 1 wherein the compound is 2′-deoxy-2′,2′-difluorocytidine.

European Patent Office

# EUROPEAN SEARCH REPORT

Application Number

EP 89 11 0278

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 211 354 (MERRELL DOW)<br>* Columns 1-4; column 11, lines 13-27 *<br>--- | 1-6 | A 61 K 31/70 |
| Y | PHARMACEUTICAL RES., no. 1, 1984, pages 11-17; R.K. ROBINS: "The potential of nucleotide analogs as inhibitors of retroviruses and tumors"<br>* Whole article *<br>--- | 1-6 | |
| Y | BIOCHEMICAL PHARMACOLOGY, vol. 36, no. 17, 1987, pages 2719-2722, Pergamon Journals Ltd, GB; V.E. MARQUEZ et al.: "2',3'-dideoxy-2'-fluoro-ara-a. An acid-stable purine nucleoside active against human immunodeficiency virus (HIV)"<br>* Whole article *<br>--- | 1-6 | |
| D,Y | EP-A-0 122 707 (ELI LILLY AND CO.)<br>* Pages 2,3; claims 21,22 *<br>--- | 1-6 | TECHNICAL FIELDS SEARCHED (Int. Cl.4) |
| P,Y | EP-A-0 287 313 (THE UNITED STATES OF AMERICA)<br>* Whole document *<br>--- | 1-6 | A 61 K<br>C 07 H |
| Y | CANCER RES., vol. 48, no. 14, 15th July 1988, pages 4024-4031; V. HEINEMANN et al.: "Comparison of the cellular pharmacokinetics and toxicity of 2',2'-difluorodeoxycytidine and 1-beta-D-arabinofuranosylcytosine"<br>* Whole article *<br>--- | 1-6 | |
| A | EP-A-0 184 365 (ELI LILLY AND CO.)<br>* Whole document *<br>--- -/- | 1-6 | |

The present search report has been drawn up for all claims

| Place of search | Date of completion of the search | Examiner |
|---|---|---|
| THE HAGUE | 07-08-1989 | FOERSTER W.K. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 11 0278

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | ANTICANCER RESEARCH, vol. 7, 1987, pages 1023-1038; E. DE CLERCQ: "Perspectives for the chemotherapy of AIDS" * Pages 1025,1030,1031 * --- | 1-6 | |
| A | CLINICAL PHARMACY, vol. 7, July 1988, pages 514-527, American Society of Hospital Pharmacists; C.J. WORDELL et al.: "Treatment of pneumocystis carinii pneumonia in patients with AIDS" * Whole document * ----- | 1-6 | |
| | | | TECHNICAL FIELDS SEARCHED (Int. Cl.4) |

The present search report has been drawn up for all claims

| Place of search | Date of completion of the search | Examiner |
|---|---|---|
| THE HAGUE | 07-08-1989 | FOERSTER W.K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document